# EUROPEAN PATENT SPECIFICATION

(11) **EP 3 082 904 B1**
(45) Date of publication and mention of the grant of the patent: **27.03.2019**
(21) Application number: 14872457.8
(22) Date of filing: 16.12.2014
(51) Int. Cl.: A61M 5/142

(54) **DOOR MOUNT STABILIZATION SYSTEM FOR AN INFUSION PUMP**
TÜRMONTAGESTABILISIERUNGSSYSTEM FÜR EINE INFUSIONSPUMPE
SYSTÈME DE STABILISATION POUR POMPE À PERFUSION PAR MONTAGE SUR PORTE

(30) Priority: 16.12.2013 US 201361916463 P; 17.12.2013 US 201361917239 P
(43) Date of publication of application: 26.10.2016
(73) Proprietor: ICU Medical, Inc., San Clemente, CA 92673 (US)
(72) Inventor: BALTEANU, Vlad R., Lake Bluff, Illinois 60044 (US); JOHNSON, Thomas D., Gurnee, Illinois 60031 (US)
(74) Representative: Invent Horizon IP
(86) International application number: PCT/US2014/070641
(87) International publication number: WO 2015/095213

(56) References cited:
- US-A- 6 110 410
- US-A- 6 110 410
- US-A1- 2012 130 341
- US-A1- 2012 145 616
- US-A1- 2012 271 226
- US-A1- 2013 177 455
- US-B1- 6 478 065
- US-B2- 8 147 448

## Description

### BACKGROUND OF THE INVENTION

The present invention is directed to a door mount stabilization system and more particularly a door mount stabilization system for an infusion pump.

Door mount systems for infusion pumps are known in the art, such as US 6,110,410, US 2012/0271226 A1, US 2012/0130341 A1 or US 2012/0145616 A1. As flow rates and demands for accuracy have increased, problems with the door mount system have resulted. More specifically, as the pump actuator force has increased, the pump chamber actuation force has been insufficient to counteract the actuator force resulting in cassette body movement during pumping with a plunger. When the cassette body moves, flow efficiency is lowered, fluid output volume varies, and limitations are created with respect to pump flow rates and low flow continuity. Therefore, a need exists in the art for a system that addresses these deficiencies.

An objective of the present invention is to provide a door mounting system that increases overall pump system stiffness.

Another objective of the present invention is to provide a door mount system that provides sufficient pump chamber rigidity to handle higher pump actuator forces.

A still further objective of the present invention is to provide a door mount system that increases flow efficiency, stabilizes output volumes, and allows for greater pump flow rates and continuity.

These and other objectives will be apparent to one of ordinary skill in the art based upon the following written description, drawings, and claims.

### BRIEF SUMMARY OF THE INVENTION

The invention is defined in the independent claim 1.

The pump has a moveable plunger that is connected to a ball screw that is operatively connected to a stepper motor. The mounting system provides sufficient strength and stability to handle increased plunger actuator force resulting in limited cassette movement within the door during pumping.

### BRIEF DESCRIPTION OF THE DRAWINGS

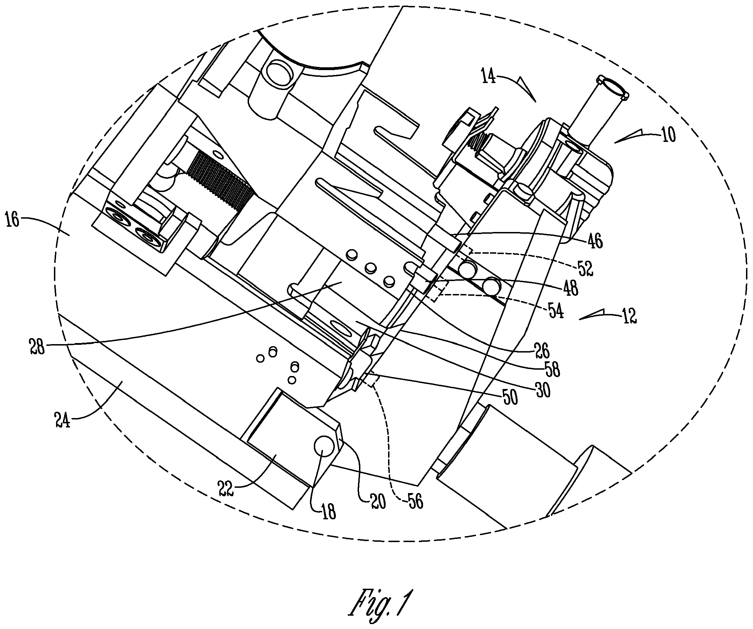

Fig. 1 is a partial side perspective view of a door mounting system;

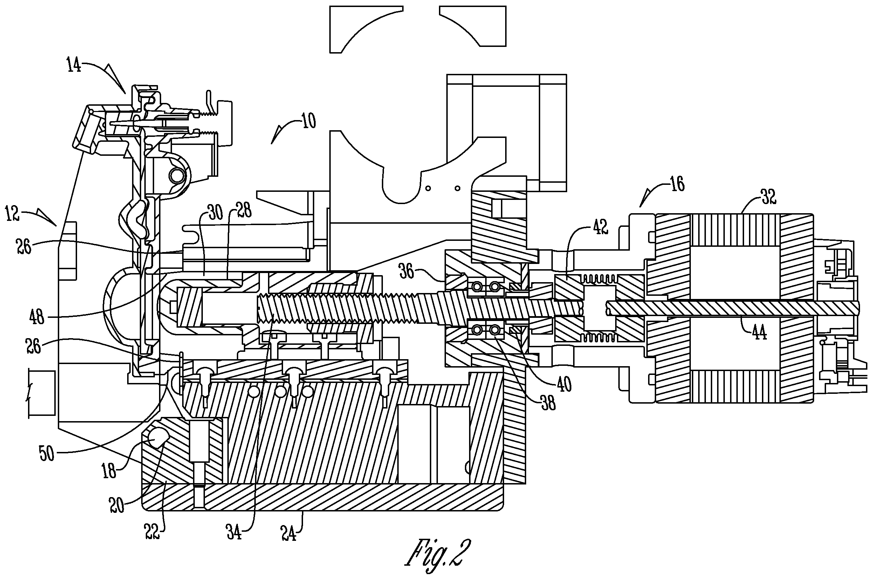

Fig. 2 is a side sectional view of a door mounting system; and

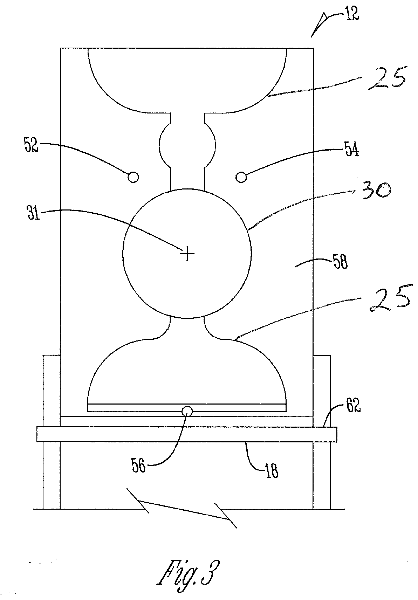

Fig. 3 is a rear view of a door in a door mounting system.

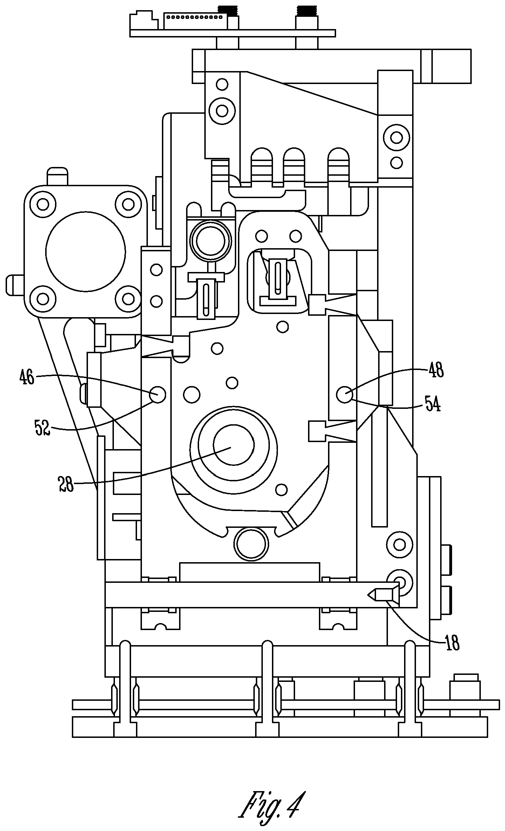

Fig. 4 is a front view of a door in a door mounting. system.

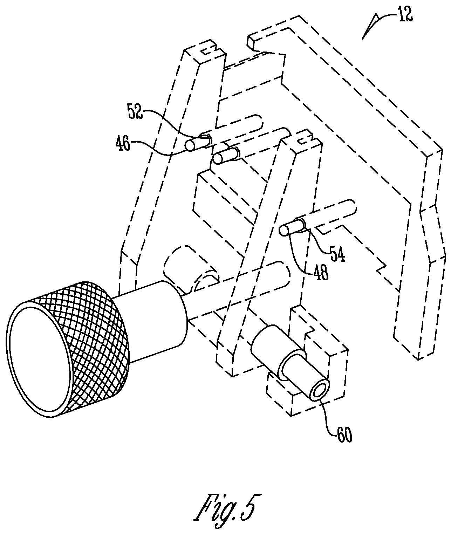

Fig. 5 is a perspective view of a door in a door mounting system.

### DETAILED DESCRIPTION OF THE PREFERRED EMBODIMENT

Referring to the Figures, the door mount stabilization system 10 includes a door 12 formed to receive a fluid administration set 14 such as a cassette or the like. The door 12 is pivotally attached to an infusion pump 16 by a pivot pin 18. The pivot pin 18 extends through a pivot pin opening 20 in a mounting block 22 that is secured to the base 24 of the infusion pump 16. The door 12 has a cavity 25 formed therein that receives a portion of the fluid administration set 14.

The infusion pump 16 has a front mechanism plate 26. Extending outwardly from the front mechanism plate 26 is a moveable plunger 28 that is aligned with and received by plunger opening 30. The plunger opening 30 has a centerline 31 that is coincident with the centerline of the cavity 25. Preferably, to improve flow performance of the pump 16, the plunger 28 is connected to a stepper motor 32 with a ball screw 34 that extends through a rotary nut 36, angular contact bearings 38, and a spacer 40 to a flexible coupler 42 that also receives a motor shaft 44. Such an arrangement actuates fluid in a gentle and smooth manner in order to keep flow laminar, keep pressure differentials to a minimum, minimize fluid shear stresses, and to smooth the flow profile.

Also, extending outwardly from the front mechanism plate 26 are first 46, second 48, and third 50 mounting pins. The mounting pins 46, 48, and 50 are positioned to align and be received by a first pin opening 52, a second pin opening 54, and a third pin opening 56 on a front face 58 of the door 12. Alternatively, but not claimed, the pins may extend from the front face 58 of the door and may be received by pin openings in the front mechanism plate 26. The first and second pins 46 and 48 are positioned above the plunger opening 30, and the third pin 50 is located below the plunger opening 30.

In one embodiment, the first and second pins 46 and 48 are located an equal distance from the centerline 31 of the plunger opening 30 and cavity 25, but spaced in opposite directions from a vertical plane that includes the centerline 31. Preferably, the first pin 46 and second pin 48 are located an equal distance from the vertical plane through the centerline 31 on a horizontal line spaced above the plunger opening 30.

In another embodiment, the third pin 50 is located on a vertical line that extends through the centerline 31 of the plunger opening 30. Preferably, the third pin 50 is located the same distance from the centerline 31 of the plunger opening 30 and cavity 25 as the first and second pins 46 and 48.

Preferably, the depths of the pin openings 52, 54, and 56 with respect to the front face 58 of the door 12 are identical and the pins 46, 48, and 50 extend outwardly from the front mechanism plate 26 the same distance or length. Alternatively, the pins 46, 48, and 50 can be of differing lengths and the corresponding openings 52, 54, and 56 can be constructed with corresponding depths to provide full engagement.

The free ends of the three pins 46, 48, and 50 engage the bottom of the pin openings 52, 54, and 56 to provide increased stiffness and even load distribution to support a cassette 14 during the pumping cycle. The stiffness is maintained in a flow range between 0.1 ml/hr to 2,000 ml/hr. The large dynamic range is achieved through use of fine motor control movements and reduced mechanical compliance. To achieve the dynamic range the pump chamber reaction force to the pump actuator force requires a 5 to 1 ratio.

In operation, the pivot pin 18 is inserted through the pivot pin opening 20 in mounting block 22 and through an opening 60 in the bottom of the door 12. The door 12 is pivoted to a closed position such that pins 46, 48, and 50 are received within openings 52, 54, and 56. Door lock 62 is engaged to maintain door 12 in a closed position. Once locked, motor 32 is activated causing plunger 28 to engage cassette 14 causing fluid to flow.

Thus a door mount stabilization system has been disclosed that, at the very least, meets all the stated objectives.

## Claims

1. A door mount system, (10) comprising:
an infusion pump (16) having a front mechanism plate; (26)
a door (10) formed to receive a fluid administration set that is pivotally connected to the infusion pump; and
a plurality of mounting pins (46, 48, 50) that extend outwardly from the front mechanism plate and are aligned with and received within openings (52, 54, 56) in a front face of the door,
**characterized in that** a first pin (46) and a second pin (48) are positioned above a plunger opening (30) in the front mechanism plate and a third pin (50) is positioned below the plunger opening,
wherein the third pin is located on a vertical line that extends through a centerline of the plunger opening, and
wherein the first and second pins are located on opposite sides of the vertical line.

2. The system of claim 1, wherein there are three mounting pins.

3. The system of claim 1, wherein the door's stiffness is maintained in a flow range between 0.1 ml/hour and 2,000 ml/hour.

4. The system of claim 1, wherein a ratio between an actuator force of the pump to a pump chamber reaction force of the door is one to five.

5. The system of claim 1, wherein the infusion pump has a plunger connected to a ball screw that is operatively connected to a stepper motor.

6. The system of claim 1, wherein the first and second pins are located an equal distance from the centerline of the plunger opening in opposite directions from a vertical plane that includes the centerline and a horizontal line spaced above the plunger opening.

7. The system of claim 3 wherein the first and second pins are located an equal distance from the centerline of the plunger opening in opposite directions from a vertical plane that includes the centerline and a horizontal line spaced above the plunger opening.

## Patentansprüche

1. Türmontagesystem (10), umfassend:
eine Infusionspumpe (16) mit einer vorderen Mechanismusplatte (26)
eine Tür (10), die zur Aufnahme eines Fluidverabreichungssets ausgebildet ist, das schwenkbar mit der Infusionspumpe verbunden ist; und
eine Vielzahl von Montagestiften (46, 48, 50), die sich von der vorderen Mechanismusplatte nach außen erstrecken und in Öffnungen (52, 54, 56) in einer Vorderseite der Tür in Flucht aufgenommen sind,
**dadurch gekennzeichnet, dass**
ein erster Stift (46) und ein zweiter Stift (48) über einer Kolbenöffnung (30) in der vorderen Mechanismusplatte positioniert sind und ein dritter Stift (50) unter der Kolbenöffnung positioniert ist,
wobei der dritte Stift auf einer vertikalen Linie angeordnet ist, die sich durch eine Mittellinie der Kolbenöffnung erstreckt, und
wobei der erste und der zweite Stift auf gegenüberliegenden Seiten der vertikalen Linie angeordnet sind.

2. System nach Anspruch 1, wobei drei Befestigungsstifte vorhanden sind.

3. System nach Anspruch 1, wobei die Steifigkeit der Tür in einem Strömungsbereich zwischen 0,1 ml/Stunde und 2000 ml/Stunde aufrechterhalten wird.

4. System nach Anspruch 1, wobei ein Verhältnis zwischen einer Stellkraft der Pumpe zu einer Pumpenkammerreaktionskraft der Tür eins zu fünf ist.

5. System nach Anspruch 1, wobei die Infusionspumpe einen Kolben aufweist, der mit einer Kugelrollspindel verbunden ist, die funktionsfähig mit einem Schrittmotor verbunden ist.

6. System nach Anspruch 1, wobei der erste und der zweite Stift in gleichem Abstand von der Mittellinie der Kolbenöffnung in entgegengesetzten Richtungen von einer vertikalen Ebene, die die Mittellinie beinhaltet, und einer horizontalen Linie, die über der Kolbenöffnung beabstandet ist, angeordnet ist.

7. System nach Anspruch 3, wobei der erste und der zweite Stift in gleichem Abstand von der Mittellinie der Kolbenöffnung in entgegengesetzten Richtungen von einer vertikalen Ebene, die die Mittellinie beinhaltet, und einer horizontalen Linie, die über der Kolbenöffnung beabstandet ist, angeordnet sind.

## Revendications

1. Système de montage sur porte (10), comprenant :
une pompe d'infusion (16) présentant une plaque de mécanisme avant (26)
une porte (10) formée pour recevoir un ensemble d'administration de fluide qui est raccordé de manière pivotante à la pompe d'infusion ; et
une pluralité de broches de montage (46, 48, 50) qui s'étendent vers l'extérieur depuis la plaque de mécanisme avant et sont alignées avec des ouvertures (52, 54, 56) dans une face avant de la porte et reçues dans celles-ci,
**caractérisé en ce qu'**une première broche (46) et une deuxième broche (48) sont positionnées au-dessus d'une ouverture de plongeur (30) dans la plaque de mécanisme avant et une troisième broche (50) est positionnée sous l'ouverture de plongeur,
dans lequel la troisième broche est située sur une ligne verticale qui s'étend au travers d'une ligne centrale de l'ouverture de plongeur, et
dans lequel les première et deuxième broches sont situées sur des côtés opposés de la ligne verticale.

2. Système selon la revendication 1, dans lequel il y a trois broches de montage.

3. Système selon la revendication 1, dans lequel la rigidité de la porte est maintenue dans une plage de flux entre 0,1 ml/heure et 2 000 ml/heure.

4. Système selon la revendication 1, dans lequel un rapport entre une force d'actionneur de la pompe et une force de réaction de chambre de pompe de la porte est de un à cinq.

5. Système selon la revendication 1, dans lequel la pompe d'infusion présente un plongeur raccordé à une vis à billes qui est raccordée en fonctionnement à un moteur pas-à-pas.

6. Système selon la revendication 1, dans lequel les première et deuxième broches sont situées à une distance égale de la ligne centrale de l'ouverture de plongeur dans des directions opposées depuis un plan vertical qui inclut la ligne centrale et une ligne horizontale espacée au-dessus de l'ouverture de plongeur.

7. Système selon la revendication 3, dans lequel les première et deuxième broches sont situées à une distance égale de la ligne centrale de l'ouverture de plongeur dans des directions opposées depuis un plan vertical qui inclut la ligne centrale et une ligne horizontale espacée au-dessus de l'ouverture de plongeur.
